# EUROPEAN PATENT APPLICATION

(11) **EP 1 430 860 A1**
(43) Date of publication of application: **23.06.2004**
(21) Application number: 02800261.6
(22) Date of filing: 27.09.2002
(51) Int. Cl.: A61F 13/04, A61L 15/12, A61L 15/14

(54) **WATER-HARDENING FIXING MATERIAL AND METHOD FOR USING THE SAME**

(30) Priority: 28.09.2001 JP 2001299812
(71) Applicant: Alcare Co., Ltd., Sumida-ku, Tokyo 131-0046 (JP)
(72) Inventor: MATSUMOTO, Yoshikazu, Alcare Co., Ltd., Sumida-ku, Tokyo 131-0046 (JP); OHNISHI, Shozo, Alcare Co., Ltd., Sumida-ku, Tokyo 131-0046 (JP)
(74) Representative: Klingseisen, Franz
(86) International application number: PCT/JP2002/010019
(87) International publication number: WO 2003/028602

(57) **Abstract**

It is an object to obtain a water-curable fixture which can be applied to even an affected part of complicated shape with sufficient time, when it is applied by winding it around an affected part such as bone fracture for fixing.

A fixture comprising a substrate and a water-curable polyurethane prepolymer composition held on the substrate, is as it is applied to an affected part. Sufficient time can be taken for application of the fixture. After completion of the application, the polyurethane prepolymer is rapidly cured by supplying water from the outside of the fixture to obtain a sufficient strength.

The substrate of the fixture has 15 to 33 apertures per cm² and a numerical aperture of 14 to 35%, and is flexible. This substrate is covered with a water-curable polyurethane prepolymer composition comprising a polyurethane prepolymer containing a polyol and polyisocyanate, and a catalyst. In this polyurethane resin composition, an ethyleneoxide component of the polyol is contained in an amount of 12wt% or higher.

## Description

### TECHNICAL FIELD

The present invention relates to a water-curable fixture useful for treatment in surgery and orthopedic surgery fields by fixing an affected part, and a method for using it.

### BACKGROUND

Water-curable fixtures obtained by coating a water-curable polyurethane prepolymer composition on a substrate of a tape-like or sheet-like shape and sealed in a sealed moisture-impermeable container, are commercially available. By immersing the water-curable fixture in water before use and applying it to an affected part, it undergoes curing in a short time, whereby the affected part can be fixed and sufficient strength can be obtained. Further, when it is used, no special equipment is required and no contamination or odor is generated. Furthermore, from the viewpoint of patients, fixing in a short time is possible, air permeability is good and roentgenography can be made, whereby it has been widely used in place of plaster bandages.

When the water-curable fixture is immersed in water to bring it into contact with water, the polyurethane prepolymer composition starts a curing reaction. When this water-curable fixture is applied to an affected part, the flexible water-curable fixture gradually hardens. When the curing reaction further proceeds, it possesses a strength such that it undergoes no deformation even if a load is applied to some extent, and when the reaction is completed, it becomes a fixture having a high strength. Since the water-curable fixture for orthopedic surgery is required to cure early after the application, the water-curable polyurethane prepolymer composition is required to have a high activity to moisture contents.

The stage of from immersing the water-curable fixture in water to attaching it to affected part comprises:
(1) a step of applying the water-curable fixture which has been brought into contact with water to an affected part (hereinafter this time zone is referred to as working life),
(2) a step of modifying the water-curable fixture partially so as to make it suited to the affected part i.e. conduct a so-called modeling (hereinafter this time zone is referred to as modeling time),
(3) a step wherein after the completion of the modeling, the curing of the water-curable fixture proceeds until deformation is no longer seen even if a load is much more applied (hereinafter this time zone is referred to as working life under load), and
(4) a step wherein the curing of the water-curable fixture further proceeds until it reaches a completely cured state.

Having considered the relation between the strength of the water-curable fixture and the time in the above steps, the step (1) is an operation of applying the water-curable fixture to the affected part, and in a case where it is applied to a site having a particularly complicated shape, it is necessary to take a time to some extent. In this step, if the curing speed of the water-curable polyurethane prepolymer composition is overly high, it is impossible to apply it appropriately to the predetermined position. Accordingly, in the time zone necessary for the application, it is desirable that the strength of the water-curable fixture is kept as low as possible.

Next, in the modeling step (2), the water-curable fixture applied to the substantially adequate position in the step (1) is partially modified to fit it into the affected part. If the strength of the water-curable fixture is kept at a low level, when a force is applied to the water-curable fixture for modification so as to conduct modeling into an accurate shape, and then the force is removed, it returns to the shape before the modification due to the restoring force or elasticity of the water-curable fixture. And, in such a case, time and labor cost are substantial for final modeling. Accordingly, it is desirable that the strength of the water-curable fixture gradually increases in the modeling step.

In the step (3), the water-curable fixture after completion of the modeling is placed at an appropriate position throughout the affected part. It is therefore required to hold the affected part so that the affected part does not move until the water-curable fixture further cures and undergoes no deformation even if usual load is applied. Accordingly, it is desirable that the strength of the water-curable fixture increases as speedily as possible (hereinafter the beginning of the curing until this step is referred to as initial curing property).

Further, in the step (4), it is desirable that, in order to shorten the binding hour of patients and doctors, the water-curable fixture cures sufficiently and the strength of the cured fixture reaches a level as high as possible.

Namely, throughout the steps (1) to (4), it is desirable that the water-curable fixture has a curing reaction property such that the strength is kept at a low level during a handling period in which it is applied to the affected part, increases promptly when it enters the modeling hour, and thereafter increases while continuously undergoing the curing, and finally reaches a high level.

Various water-curable fixtures which have been proposed in the prior art are, as mentioned above, ones which are immersed in water at the time of use to let them hold water sufficiently, and then are wound around the affected part for application. As mentioned above, it is required to speed up the curability of the polyurethane prepolymer composition after the modeling step. If doing so, however, the curing steadily proceeds even during the application to the affected part, whereby the working life is shortened and it is difficult to apply it with sufficient time to complicated affected part, such being disadvantageous.

### SUMMARY OF THE INVENTION

It is an object of the present invention to provide a water-curable fixture which can be applied to even an affected part of complicated shape with sufficient working life, and accelerates the curing property when it enters the modeling hour, and is easily used and finally presents a sufficient strength.

In the present invention, a water-curable fixture comprising a substrate and a water-curable polyurethane prepolymer composition held on the substrate, is as it is applied to an affected part. After completion of the application, the prepolymer is rapidly cured by supplying water from the outside of the fixture to obtain a sufficient strength. At this time, the substrate of the fixture has 15 to 33 apertures per cm² and a numerical aperture of 14 to 35%, and is flexible. This substrate is covered with a water-curable polyurethane prepolymer composition comprising a polyurethane prepolymer containing a polyol and polyisocyanate, and a catalyst. In this polyurethane resin composition, an ethylene oxide component of the polyol is contained in an amount of 12wt% or higher.

The water content to be supplied after the water-curable fixture is applied to the affected part, may be water solely, but if water containing a surfactant is used, the water content can be evenly applied throughout the fixture, and the curing can be accelerated. Further, it is advantageous to supply the water content by spraying it over the fixture applied to the affected part.

In order to accelerate the curability after the start of the modeling step, it is desirable that the polyurethane prepolymer composition appropriately generates heat. For this purpose, a compound which is capable of generating heat when it contacts water may be added to the polyurethane polymer composition. In such a case, when the modeling step operation is carried out by supplying water to the fixture applied to the affected part, the contact of this heat-generating compound with water can be further easily caused at the outermost layer of the fixture. And, since the heat is supplied from the outermost layer toward the inner layer of the fixture, the curing can further be accelerated.

### BRIEF EXPLANATION OF THE DRAWINGS

Fig. 1 is a table showing materials used for the water-curable polyurethane prepolymer composition in examples and comparative examples of the present invention.
Fig.2 is a table showing the formulation of the water-curable polyurethane prepolymer composition in examples of the present invention.
Fig.3 is a table showing the formulation of the water-curable polyurethane prepolymer composition in comparative examples of the present invention.
Fig.4 is a table showing the results of the respective measurements in examples of the present invention.
Fig.5 is a table showing the results of the respective measurements in comparative examples of the present invention.
Fig.6 is a graph showing the relation between the initial curability and the elapsed time in examples and comparative examples of the present invention.

### BEST MODE FOR CARRYING OUT THE INVENTION

As the substrate for the water-curable fixture, it is possible to use knit, woven fabric, unwoven fabric and the like obtained from materials which are flexible, have a low moisture content and a high tensile strength, and are inactive to the polyurethane prepolymer composition and easily wettable. For example, knit, woven fabric and unwoven fabric using glass fibers, aramid fibers, polyester fibers, polyolefin fibers, polyamide fibers, polyacryl fibers, rayon fibers, cotton fibers and the like, may be mentioned. Raschel fabric using yarn of glass fibers or polyester fibers is particularly preferred, and the thickness is preferably about 0.08 to 5mm.

This substrate preferably has about 15 to 33 apertures per cm² and a numerical aperture of about 14 to 35%. With apertures having the number of apertures of 34 or more per cm² and the numerical aperture of less than 34%, the permeability of water applied by e.g. spraying is low. With apertures having the number of apertures of 14 or less per cm² and the numerical aperture exceeding 35%, the water applied by e.g. spraying is not held and flows out. Accordingly, in both cases, it is difficult to cure the polyurethane prepolymer composition sufficiently. The numerical aperture is the ratio of the area occupied by the spacing in a constant area, and can be determined mechanically by taking an enlarged photograph of the substrate and making image recognition of the photograph.

The polyurethane prepolymer is the one which is obtained by the reaction of a polyol with polyisocyanate and has an isocyanate group at its terminal end.

As the polyol, a random or block copolymer of ethylene oxide and propylene oxide, and polyethylene glycol (PEG) are used. The number average molecular weight of the polyol is desirably about 200 to 4,000. If the molecular weight is less than 200, the rigidity is large, leading to hard and brittle properties. If the molecular weight is higher than 4,000, the rigidity is small, the strength of the fixture is sometimes insufficient. Further, together with these polyols, known polyols can be used. For example, polypropylene glycol (PPG), bisphenol type diol (BP, BPE) and the like may be mixed appropriately for use. It is necessary that the ethylene oxide component of the polyol is contained in an amount of about 12wt% or higher, preferably about 18wt% or higher, in the polyurethane prepolymer composition.

As the polyisocyanate, well known polyisocyanate may be used. For example, 4,4'-diphenylmethane diisocyanate, 2,4'-diphenylmethane diisocyanate, polymethylenepolyphenyl polyisocyanate, or the like, and p-phenylene diisocyanate and carbodiimide-modified polyisocyanate of them may be mentioned. These may be used alone or in combination of two or more of them. It is preferred to use 4,4'-diphenylmethane diisocyanate, 2,4'-diphenylmethane diisocyanate, and carbodiimide-modified polyisocyanate of them.

As the proportional ratio of a polyol and polyisocyanate to obtain the polyurethane prepolymer having an isocyanate group at its terminal end, the polyisocyanate is usually 2 to 5 equivalents, preferably 2.5 to 5 equivalents, per equivalent of the polyol. The reaction of both is made by heating and stirring usually at about 30 to 100□, preferably at about 50 to 80□. The viscosity of the polyurethane prepolymer is usually about 10 to 50 Pa·s, preferably about 15 to 40 Pa·s at room temperature of 23□.

As the catalyst, the one excellent in storage stability should preferably be selected for use. As the ones well known in the prior art, dimorpholino diethylether, bis(2,6-dimethylmorpholino)diethylether, and substituted morpholino diethylether may, for example, be mentioned. These may be used alone or in combination of two or more of them.

After the application of the water-curable fixture to the affected part, when curing is conducted by spraying water, the amount of the catalyst is preferably such that the after-mentioned gelling time of the polyurethane prepolymer composition is 70 seconds or less. For this purpose, the catalyst is preferably contained in an amount of about 1.3 to 6wt% in the composition. If the catalyst amount is less than 1.3wt%, in the above-mentioned initial curability, the curability after spraying water can not sufficiently be improved, and within a range of the catalyst amount exceeding 4wt% until 6wt%, the strength is not changed as compared with the initial curability when the catalyst amount is 4wt%, and if it exceeds 6wt%, the strength lowers.

In the polyurethane prepolymer composition, a compound which is capable of generating heat when it contacts water (dissolution) may be contained. As the compound which is capable of generating heat when it contacts water, various types may be used. For example, chlorides of metals such as calcium chloride and magnesium chloride, oxides of metals such as calcium oxide and zinc oxide, sulfates of metals such as calcium sulfate and magnesium sulfate, silica and the like may be mentioned.

In the polyurethane prepolymer composition, a stabilizer may appropriately be contained. As the stabilizer, well known benzoyl chloride, methanesulfonic acid, p-toluenesulfonic acid may be used. These stabilizers may likewise be used alone or in combination of two or more of them. The amount of the stabilizer is determined depending upon the catalyst amount, and usually about 0.005 to 1wt%, preferably about 0.01 to 0.5wt% of the polyurethane prepolymer composition. When it is less than 0.005wt%, stabilization effect may not be obtained, and when it exceeds 1wt%, the activity of the catalyst may be impaired.

In the polyurethane prepolymer composition of the present invention, as the case requires, a defoaming agent, an antioxidant, a viscosity modifier, an adhesion retardant, an ultraviolet absorber, colorants such as a pigment and a dye, fillers such as calcium carbonate, titanium dioxide, carbon black, clay and the like, may further be contained.

As the amount of the water to be supplied to the water-curable fixture by spraying or the like, the curing can be made by supplying a small amount at a level of about 20 to 60 g/m², preferably at a level of about 40 to 50 g/m². Further, it is preferred to add a surfactant to the water to be supplied. As the surfactant, any one of nonionic, anionic, cationic and ampholytic surfactants may be used, but ones having a low epispastic property is desirable. For example, as the nonionic type, polyoxyethylene alkylether type, and as the anionic type, sodium polyoxyethylene laurylether sulfate, polyoxyethylene laurylether sulfate triethanolamine and the like are preferred. As the specific spraying method, the above predetermined amount is sprayed by a sprayer or an aerosol container filled with water containing the surfactant or containing no surfactant.

For preparation of the polyurethane prepolymer composition, the catalyst, stabilizer and various additives may be added to the polyurethane prepolymer obtained from a polyol and polyisocyanate. Further, at the time of preparing the polyurethane prepolymer, together with the polyol and polyisocyanate, a part or whole of a catalyst, a stabilizer, calcium chloride and various additives may preliminarily be added. Further, after polyurethane prepolymer is coated on a substrate cloth, calcium chloride may be sprinkled and adhered on it.

The method for producing the water-curable fixture by covering the substrate with the polyurethane prepolymer composition, may be conducted by well known methods. For example, a method wherein the polyurethane prepolymer composition is coated on the substrate by a roll in a room controlled at a low humidity. The obtained water-curable fixture is desirably kept in a sealed container which is capable of blocking moisture.

When the water-curable fixture is used, the sealed container is opened and the fixture is taken out, and this fixture is as it is applied by winding it around the affected part. At this time, since water is not yet applied to the fixture, full-scale curing is not yet started although a slight curing might have been started by the moisture in air. Accordingly, it is possible to securely apply it with sufficient time even to an affected part of complicated shape.

After it is applied as mentioned above, by applying water content by spraying water on the surface of the fixture, polyurethane prepolymer starts the reaction and undergoes curing. When a heat-generating material such as calcium chloride is contained in the fixture, the reaction is accelerated by further heat generation. When modeling is conducted in the progress of curing to make modification so as to fit it in the affected part, and the curing is further advanced, the affected part can be securely fixed.

The process of the curing of the water-curable fixture is considered as follows. Namely, polyethylene glycol as the polyol content in the polyurethane prepolymer has a strong hydrophilicity. After the fixture is applied to the affected part, when water is sprayed from the outside thereof, the water content rapidly impregnates into the polyurethane prepolymer composition and starts the reaction with the isocyanate group. At this time, if a surfactant is contained in the water to be sprayed, the permeability into the water-curable fixture is increased and the water can be applied more evenly throughout it. Further, the permeability of water is also related to the above-mentioned number of apertures and numerical aperture. Within a range where the number of apertures is 14 to 34 per cm² of the substrate and the numerical aperture is 13 to 35%, excellent results can be obtained. Preferably, the number of apertures is 15 to 33 and the numerical aperture is 14 to 33%, more preferably, the number of apertures is 20 to 25 and the numerical aperture is 18 to 27%.

If water impregnates and is held in the fixture effectively, the catalyst in the polyurethane prepolymer composition accelerates the reaction of water with isocyanate to advance the curing. By the reaction heat by the reaction of water with isocyanate, the temperature is raised and the reaction rate is further increased, whereby the curing can be made from the outer layer toward the inner layer at the affected part side in a short time. And, since the ratio that water reaches the most affected part side (most inner side) of the fixture is low, the heat generation at the affected part side is small.

When the curing is made by a spray method, with a polyurethane prepolymer using polypropylene glycol, the hydrophilicity is low and the permeability of water is small, whereby it is difficult to obtain the desired strength in a short time at a practical level. Accordingly, in order to obtain a preferred water permeability, the ethylene oxide component as the polyol is contained in an amount of 12wt% or higher, preferably 18wt% or higher in the polyurethane prepolymer composition. Since such polyurethane prepolymer has a high hydrophilicity, the reaction completes in an early time while generating the reaction heat. Further, for the increase of the initial curability, the catalyst amount is influential in addition to the water permeability. Accordingly, as the catalyst amount, it is preferably added to the polyurethane prepolymer composition so that the gelling time is 70 seconds or lower.

### EXAMPLES

The embodiments of the present invention will be explained further specifically with reference to examples and comparative examples. However, it should be mentioned that the present invention is by no means restricted thereto and those skilled in the art can understand various other embodiments.

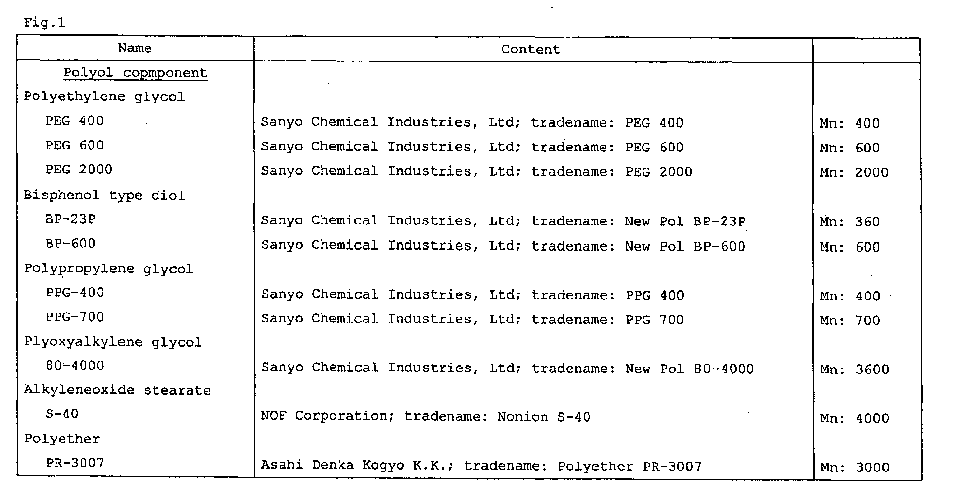

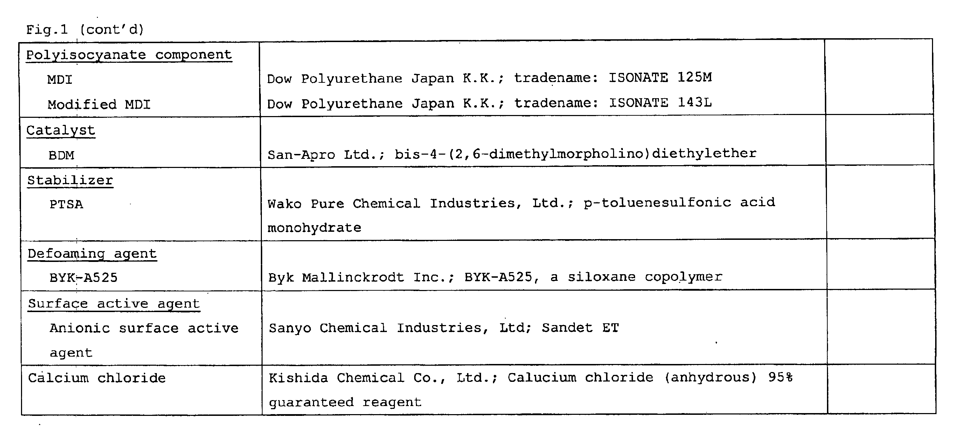

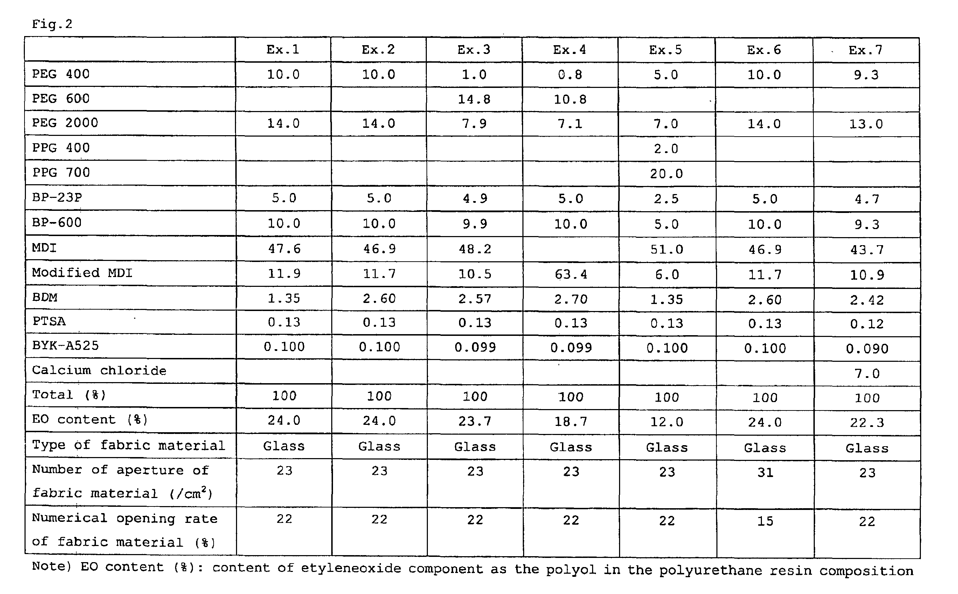

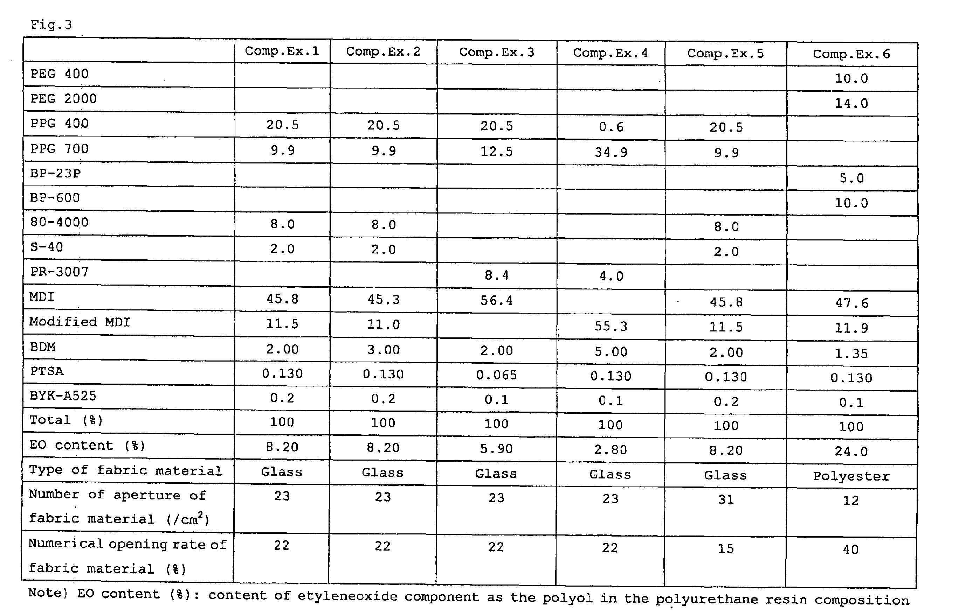

The materials used for the polyurethane prepolymer compositions of the examples and comparative examples are indicated in Fig. 1, and the formulations are indicated in Fig.2 and Fig.3. Further, the physical properties of the polyurethane prepolymer compositions obtained from these formulations and the water-curable fixtures coated on the substrate were evaluated with respect to the following items.

In preparation of the polyurethane prepolymer compositions, in all of the examples and comparative examples, a polyol component and a defoaming agent were charged into a reaction container flushed with nitrogen gas, and the water content was removed at 80 to 1000, and then a part of a stabilizer was added. Then, a polyisocyanate component was added thereto, and stirring was conducted at 70 to 80□ for about 3 hours. Further, a catalyst and the rest of the stabilizer were added, and stirring was conducted for one hour to obtain a polyurethane prepolymer composition. This polyurethane prepolymer composition was sealed in a container which was flushed with nitrogen gas.

In all of the examples and comparative examples, preparation of the water-curable fixture was conducted by coating the composition on a substrate. As the substrate, a tape-shaped substrate obtained by raschel fabrication of glass fibers to have a number of apertures (mesh size) of 23 per cm², a numerical aperture of 22% and a width of 100mm, and further heat-cleaning, was used. Further, one which was the same as above provided that the number of apertures of 31 per cm² and the numerical aperture of 15%, was used. Further, in one of the comparative examples, a tape-shaped substrate obtained by raschel fabrication of polyester fibers under the conditions that a number of apertures (mesh size) of 12 per cm² and a numerical aperture of 40%, and heat-setting, was used.

The coating of the polyurethane prepolymer composition on this substrate was carried out by a roll coater method. The polyurethane prepolymer composition was coated on the glass substrate in 220 g/m² on average. Further, it was coated on the polyester substrate in 280 g/m² on average. The substrate on which the polyurethane prepolymer composition was coated was wound up in a length of 3.6 m to obtain a water-curable fixture, and sealed in a moisture-impermeable bag which was flushed with nitrogen gas.

Evaluation of the physical properties of the polyurethane prepolymer compositions and the water-curable fixtures were conducted as follows, respectively. In a spray method, unless otherwise specifically described, a 1 wt% aqueous solution having an anionic surfactant i.e. polyoxyethylene laurylether sulfate triethanolamine (manufactured by Sanyo Chemical Industries, Ltd; "Sandet ET") added to water, was used. As the spray method, spraying was made by a sprayer so that the composition would be attached uniformly in an amount of 40g to 1m² of the water-curable fixture.

The evaluation of the water-curable fixtures was made with respect to the following items.

### Working life in an immersion method

In a measurement room controlled to have a room temperature of 23□ and a humidity of 65%RH, the water-curable fixture was taken out of the moisture-impermeable bag, and immersed in water of 20□ for 10 seconds, and lightly drained. This water-curable fixture was wound around the outer periphery of a cylinder, and the time when rolling can no longer be made was measured.

### Working life in a spray method

In a measurement room controlled to have a room temperature of 23□ and a humidity of 65%RH, the water-curable fixture was taken out of the moisture-impermeable bag, and wound as it is around the outer periphery of a cylinder, and the time when rolling can no longer be made was measured.

### Heat generation temperature in a spray method

In a measurement room controlled to have a room temperature of 20□ and a humidity of 20%RH, a temperature sensor was provided on the surface of a polyethylene container of 85mm in diameter which was preliminarily heated to 37□ by hot water. On this polyethylene container, a backing material of unwoven fabric having a thickness of 3mm was wound in two layers, and further thereon, a water-curable fixture taken out of the moisture-impermeable bag was wound in 13 layers. Over the 13-layer round up fixture, water containing a surfactant was sprayed by a sprayer evenly throughout it, and the maximum heat-generation temperature was measured.

### Change of strength with the lapse of time in an immersion method (initial curability in an immersion method)

In a measurement room controlled to have a room temperature of 20D and a humidity of 20%RH, the water-curable fixture was taken out of the moisture-impermeable bag, and immersed in water of 20□ for 5 seconds. At this time, the sample was taken out without kneading it, and lightly grabbed, and swung 3 times to drain the water. Then, the water-curable fixture was wound in 5 layers without applying tension around an acryl cylindrical container on which a backing material was preliminarily wound and which was 60mm in diameter, controlled at 32D and filled with water. And, while taking a care not to let it shift in a transverse direction, the surface was rubbed to form a cylindrical sample. After 5 minutes had passed, the sample was drawn out from the acryl cylindrical container without causing deformation, and the sample was mounted on a jig having a distance of 50mm between supporting points. This sample was compressed in a radial direction by Autograph AG-D(manufactured by Shimazu Corporation; a precision universal testing machine of a computer-controlled measurement type), and the stress until deformation of 5mm was observed was measured. Likewise, after 7 minutes had passed and 10 minutes had passed, other samples prepared in the same way were measured. The test speed was 25 mm/min.

### Change of strength with the lapse of time in a spray method (initial curability in a spray method)

In a measurement room controlled to have a room temperature of 20□ and a humidity of 20%RH, the water-curable fixture taken out of the bag was wound in 5 layers without applying tension around an acryl cylindrical container on which a backing material was preliminarily wound and which was 60mm in diameter, controlled at 320 and filled with water. Over the surface of the roll, the water containing a surfactant was sprayed by a sprayer evenly, and while taking a care not to let it shift in a transverse direction, the surface was rubbed to form a cylindrical sample. After 5 minutes had passed, the sample was drawn out from the acryl cylindrical container without causing deformation, and the stress was likewise measured by Autograph AG-D. Likewise, after 7 minutes had passed and 10 minutes had passed, other samples prepared in the same way were measured.

### Compressive strength one day after

In the same manner as the above initial curability, samples were prepared for the immersion method and the spray method, and 15 minutes after, these were drawn out without causing deformation. After these were left to stand in a constant temperature oven of 20□ for one day, the stress was likewise measured by Autograph AG-D. The test speed was 25 mm/min.

### Gelling time

In a measurement room controlled to have a room temperature of 20□ and a humidity of 20%RH, 5g of a polyurethane prepolymer composition was weighed, and 1 g of water was added thereto, followed by stirring with a glass rod for 20 seconds. Then, the resin elevated while bubbling, and the time from the start of stirring until the bubble is broken at around the portion where the elevation stopped was measured and it was called a gelling time (seconds).

### Results of measurements

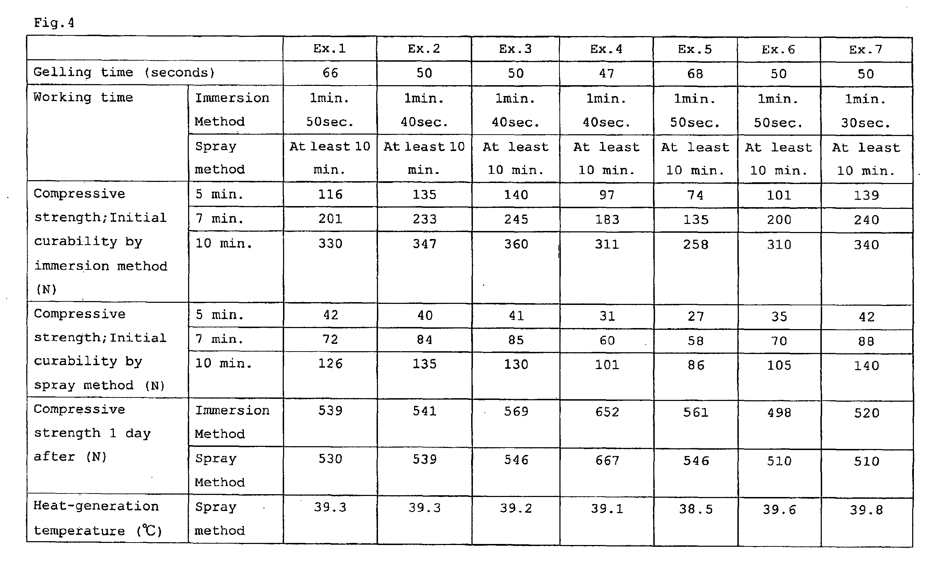

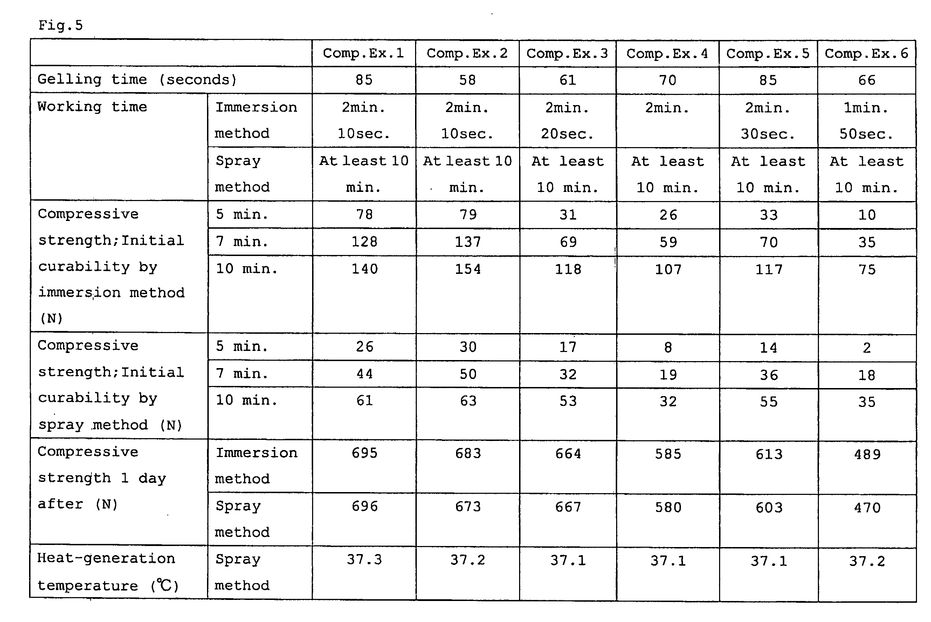

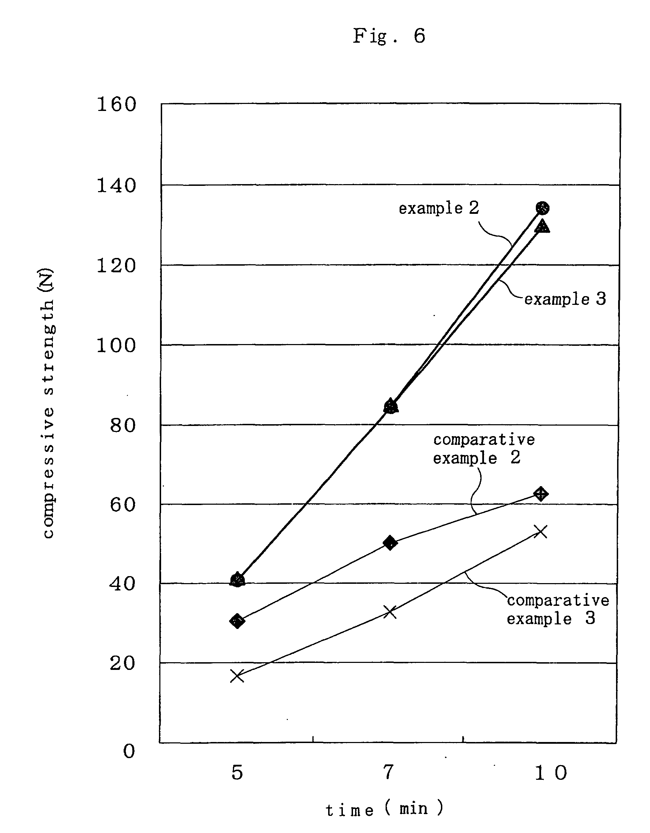

The results of respective measurements concerning the physical properties and evaluation in examples and comparative examples are indicated in Fig.4 and Fig.5. Further, the relations between the elapsed time and the strength concerning the initial curability for respective spray methods in Examples 2 and 3 and Comparative Examples 2 and 3 are indicated in Fig. 6. With respect to the above measurement results, explanation will be given below.

### Working life

In respective Examples of the present invention, since the working life by the spray method is 10 minutes or longer, it is possible to securely apply the fixture to complicated sites of the affected part with sufficient time. On the other hand, in the immersion method of Comparative Examples, since the working life is 1 minute 50 seconds to 2 minutes 30 seconds, this time is too short to apply the fixture to complicated sites and the fixture may sometimes be cured before the completion of application. Here, if the ones in Examples are applied by the immersion method, since the working life is as short as 1 minute 30 seconds to 1 minute 50 seconds, it is difficult in the immersion method to take sufficient time for the application to the affected part.

### Initial curability

It is commonly known that the initial curability is not at a practical level if the strength does not reach about 78N or higher at the point of 10 minutes later. In the ones made by the spray method of Examples, the strength is within a range of 86 to 140N i.e. over 78N, and it is confirmed that these have an appropriate initial curability.

On the other hand, the ones in Comparative Examples 1 to 5, the strength is 107 to 154N by the immersion method, such being appropriate. However, by the spray method, the strength is 32 to 63N i.e. does not reach 78N, and it is therefore concluded that the use by the spray method is inconvenient. Further, by the one in Comparative Example 6, although sufficient strength can be obtained in the after-mentioned compressive strength one day after, no sufficient initial curability can be obtained in both the immersion method and the spray method. Furthermore, in the ones in Examples, in the immersion method, the strength exceeds 78N in an early time at a level of about 5 minutes, and the curing is too fast.

### Compressive strength one day after

In Examples and Comparative Examples, sufficient compressive strength can be seen in both the spray method and the immersion method, and therefore no difference is seen in this point.

### Heat-generation temperature

In the spray method, the maximum temperature in Examples is 38.5 to 39.8□, and this heat generation is within an allowable range and no inconvenience is caused. On the other hand, in Comparative Examples, the heat-generation temperature is low, and the reaction does not proceed at an appropriate speed, and the low numerical value of the initial curability reflects this result.

### Gelling time

In respective Examples and Comparative Examples, the gelling times in Comparative Examples 1 and 5 are over 70 seconds and considered to be too long, but other ones including respective Examples are within an appropriate range.

### Total evaluation

In the ones of Examples, in the case of the spray method, sufficient working life is obtained, appropriate numerical values are obtained in the initial curability and the compressive strength one day after, and the heat-generation temperature is appropriate, whereby these can be used adequately.

In the ones of Comparative Examples, in the case of the spray method, the working life is long, but the heat-generation temperature is low, whereby no sufficient initial curability can be obtained. Further, in the case of the immersion method, it is considered that the initial curability and the compressive strength one day after are within appropriate ranges, but the working life is too short, such being problematic in use.

### INDUSTRIAL APPLLICABILITY

In the present invention, in the case where the water content is supplied by a spray method for curing, the working life is 10 minutes or longer as mentioned above. Accordingly, even when it is applied to an affected part of complicated shape, curing is not advanced in the middle of application, and sufficient time can be obtained. Further, since the water content for curing is supplied from the outside after the fixture is wound in several layers, the water content amount which reaches the affected part is small, and the inner side is not substantially wetted, whereby the heat generation at the affected part side is not so high. Accordingly, unpleasantness of patients can be reduced.

Further, since this fixture can be cured with a small amount of water by the spray method, it is not necessary to prepare a large amount of water in a bucket like the immersion method. And, if an aerosol container or the like filled with water is prepared, this fixture can be used anywhere without contaminating floors. Accordingly, it can be effectively used for treatment of e.g. bone fracture in a site where water can not be prepared easily such as disaster-stricken district.

## Claims

1. A water-curable fixture which comprises a relatively coarse and flexible substrate and a water-curable polyurethane prepolymer composition which is capable of curing in a short time by applying a small amount of water content after application to an affected part, coated on the substrate, wherein the water-curable polyurethane prepolymer composition contains a polyurethane prepolymer containing a polyol component and polyisocyanate, and a catalyst, **characterized in that** the water-curable polyurethane prepolymer composition contains an ethyleneoxide component as the polyol component in an amount of 12wt% or higher, and the substrate has 15 to 33 apertures per cm² and a numerical aperture of 14 to 35%.

2. The water-curable fixture according to Claim 1, wherein the water content applied after application to the affected part contains a surfactant.

3. The water-curable fixture according to Claim 2, wherein the surfactant has a low epispastic property.

4. The water-curable fixture according to Claim 3, wherein the surfactant is a nonionic or anionic surfactant.

5. The water-curable fixture according to Claim 1, wherein the catalyst is contained in such an amount that a gelling time of the water-curable polyurethane prepolymer composition is 70 seconds or lower.

6. The water-curable fixture according to any one of Claims 1 to 4, wherein the water-curable polyurethane prepolymer composition contains a compound which is capable of generating heat when it contacts water to accelerate the curing of the polyurethane prepolymer.

7. The water-curable fixture according to Claim 6, wherein the compound which accelerates the curing is any one of a chloride of a metal, an oxide of a metal, a sulfate of a metal, and silica.

8. The water-curable fixture according to Claim 7, wherein the chloride of a metal is any one of calcium chloride and magnesium chloride.

9. The water-curable fixture according to Claim 7, wherein the oxide of a metal is any one of calcium oxide and zinc oxide.

10. The water-curable fixture according to Claim 7, wherein the sulfate of a metal is any one of calcium sulfate and magnesium sulfate.

11. A method for using a water-curable fixture, which comprises preparing a water-curable fixture which comprises a substrate having 15 to 33 apertures per cm² and a numerical aperture of 14 to 35%, and a water-curable polyurethane prepolymer composition which contains a polyol component containing an ethyleneoxide component in an amount of 12wt% or higher and polyisocyanate, and a catalyst, coated on the substrate; winding a backing material around an affected part; winding the water-curable fixture over the backing material; and spraying water from the outside of the water-curable fixture so as to conduct curing.

12. The method for using a water-curable fixture according to Claim 11, wherein the water content sprayed contains a surfactant.

13. The method for using a water-curable fixture according to Claim 12, wherein the surfactant has a low epispastic property.

14. The method for using a water-curable fixture according to Claim 13, wherein the surfactant is a nonionic or anionic surfactant.

15. The method for using a water-curable fixture according to any one of Claims 11 to 14, wherein the sprayed water amount is 20 to 60 g/m².
